(19) Europäisches Patentamt
European Patent Office
Office européen des brevets

(11) **EP 3 897 375 B1**

(12) **EUROPEAN PATENT SPECIFICATION**

(45) Date of publication and mention
of the grant of the patent:
**23.07.2025 Bulletin 2025/30**

(21) Application number: **19899138.2**

(22) Date of filing: **17.12.2019**

(51) International Patent Classification (IPC):
**A61B 5/24** (2021.01)     **A61B 5/00** (2006.01)

(52) Cooperative Patent Classification (CPC):
**A61B 5/7203; A61B 5/24; A61B 5/4836;
A61B 5/7235;** A61B 5/0031; A61B 5/7225

(86) International application number:
**PCT/AU2019/051385**

(87) International publication number:
**WO 2020/124135 (25.06.2020 Gazette 2020/26)**

(54) **IMPROVED DETECTION OF ACTION POTENTIALS**

VERBESSERTE ERFASSUNG VON AKTIONSPOTENTIALEN

DÉTECTION AMÉLIORÉE DE POTENTIELS D'ACTION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **17.12.2018 US 201862780873 P**

(43) Date of publication of application:
**27.10.2021 Bulletin 2021/43**

(73) Proprietor: **Saluda Medical Pty Ltd
Macquarie Park NSW 2113 (AU)**

(72) Inventors:
• **PARKER, Daniel John**
  **Artarmon, NSW 2064 (AU)**
• **SINGLE, Peter Scott Vallack**
  **Artarmon, NSW 2064 (AU)**
• **HUANG, Kai**
  **Artarmon, NSW 2064 (AU)**

(74) Representative: **Abel & Imray LLP
Westpoint Building
James Street West
Bath BA1 2DA (GB)**

(56) References cited:
**WO-A1-2018/170141     US-A1- 2006 287 609
US-B2- 8 249 698     US-B2- 9 044 155
US-B2- 9 044 155**

• **ISLAM MD KAFIUL ET AL: "Methods for artifact
detection and removal from scalp EEG: A
review", NEUROPHYSIOLOGIE CLINIQUE -
CLINICAL NEUROPHYSIOLOGY, vol. 46, no. 4, 15
October 2016 (2016-10-15) - 15 October 2016
(2016-10-15), pages 287 - 305, XP029804850,
ISSN: 0987-7053, DOI: 10.1016/
J.NEUCLI.2016.07.002**
• **CHI ZHANG, LI TONG, YING ZENG, JINGFANG
JIANG, HAIBING BU, BIN YAN, JIANXIN LI:
"Automatic Artifact Removal from
Electroencephalogram Data Based on A Priori
Artifact Information", BIOMED RESEARCH
INTERNATIONAL, vol. 215, 720450, 25 August
2015 (2015-08-25), pages 1 - 9, XP055721674,
ISSN: 2314-6133, DOI: 10.1155/2015/720450**
• **MALIK MUHAMMAD NAEEM MANNAN ,
MUHAMMAD AHMAD KAMRAN , MYUNG YUNG
JEONG: "Identification and Removal of
Physiological Artifacts From
Electroencephalogram Signals: A Review", IEEE
ACCESS, vol. 6, 31 May 2018 (2018-05-31), pages
30630 - 30652, XP055721679, DOI: 10.1109/
ACCESS.2018.2842082**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 897 375 B1

## Description

<u>Technical Field</u>

The present invention is as defined in the appended claims and generally relates to electrical recording of neural activity such as compound action potentials evoked by neurostimulation, and in particular to systems and methods for improved detection of neural responses in the presence of stimulus artefact, noise and the like.

<u>Background of the Invention</u>

Electrical neuromodulation is used or envisaged for use to treat a variety of disorders including chronic pain, Parkinson's disease, and migraine, and to restore function such as hearing and motor function. A neuromodulation system applies an electrical pulse to neural tissue in order to generate a therapeutic effect. Such a system typically comprises an implanted electrical pulse generator, and a power source such as a battery that may be rechargeable by transcutaneous inductive transfer. An electrode array is connected to the pulse generator, and is positioned close to the neural pathway(s) of interest. An electrical pulse applied to the neural tissue by an electrode causes the depolarisation of neurons, which generates propagating action potentials whether antidromic, orthodromic, or both, to achieve the therapeutic effect.

When used to relieve chronic pain for example, the electrical pulse is applied to the dorsal column (DC) of the spinal cord and the electrode array is positioned in the dorsal epidural space. The dorsal column fibres being stimulated in this way inhibit the transmission of pain from that segment in the spinal cord to the brain.

In general, the electrical stimulus generated in a neuromodulation system triggers a neural action potential which then has either an inhibitory or excitatory effect. Inhibitory effects can be used to modulate an undesired process such as the transmission of pain, or excitatory effects can be used to cause a desired effect such as the contraction of a muscle or stimulation of the auditory nerve.

The action potentials generated among a large number of fibres sum to form a compound action potential (CAP). The CAP is the sum of responses from a large number of single fibre action potentials. When a CAP is electrically recorded, the measurement comprises the result of a large number of different fibres depolarising. The propagation velocity is determined largely by the fibre diameter and for large myelinated fibres as found in the dorsal root entry zone (DREZ) and nearby dorsal column the velocity can be over 60 ms$^{-1}$. The CAP generated from the firing of a group of similar fibres is measured as a positive peak P1 in the recorded potential, then a negative peak N1, followed by a second positive peak P2. This is caused by the region of activation passing the recording electrode as the action potentials propagate along the individual fibres, producing the typical three-peaked response profile. Depending on stimulus polarity and the sense electrode configuration, the measured profile of some CAPs may be of reversed polarity, with two negative peaks and one positive peak.

To better understand the effects of neuromodulation and/or other neural stimuli, and for example to provide a stimulator controlled by neural response feedback, it is desirable to accurately detect and record a CAP resulting from the stimulus. Evoked responses are less difficult to detect when they appear later in time than the artefact, or when the signal-to-noise ratio is sufficiently high. The artefact is often restricted to a time of 1 - 2 ms after the stimulus and so, provided the neural response is detected after this time window, a response measurement can be more easily obtained. This is the case in surgical monitoring where there are large distances (e.g. more than 12 cm for nerves conducting at 60 ms$^{-1}$) between the stimulating and recording electrodes so that the propagation time from the stimulus site to the recording electrodes exceeds 2 ms.

However to characterize the responses from the dorsal columns, high stimulation currents and close proximity between electrodes are required. Similarly, any implanted neuromodulation device will necessarily be of compact size, so that for such devices to monitor the effect of applied stimuli the stimulus electrode(s) and recording electrode(s) will necessarily be in close proximity. In such situations the measurement process must overcome artefact directly. However, this can be a difficult task as an observed CAP signal component in the neural measurement will typically have a maximum amplitude in the range of microvolts. In contrast a stimulus applied to evoke the CAP is typically several volts and results in electrode artefact, which manifests in the neural measurement as a decaying output of several millivolts partly or wholly contemporaneously with the CAP signal, presenting a significant obstacle to isolating or even detecting the much smaller CAP signal of interest.

For example, to resolve a 10 $\mu$V CAP with 1 $\mu$V resolution in the presence of an input 5 V stimulus, for example, requires an amplifier with a dynamic range of 134 dB, which is impractical in implant systems. As the neural response can be contemporaneous with the stimulus and/or the stimulus artefact, CAP measurements present a difficult challenge of measurement amplifier design. In practice, many non-ideal aspects of a circuit lead to artefact, and as these mostly have a decaying exponential appearance that can be of positive or negative polarity, their identification and elimination can be laborious.

The difficulty of this problem is further exacerbated when attempting to implement CAP detection in an implanted device. Typical implants have a power budget which permits a limited number, for example in the hundreds or low thousands, of processor instructions per stimulus, in order to maintain a desired battery lifetime. Accordingly, if a CAP detector for an implanted device is to be used regularly, such as of the order of once a second, then care must be taken that the detector should consume only a small fraction of the power budget. US9044155 discloses a method of processing an electrical stimulation response measurement waveform signal. A source separation algorithm is used to remove a stimulus artefact component. WO2018/170141 discloses a method of removing a stimulation artefact in a multi-channel neural recording by estimating the stimulation artifacts. ISLAM MD KAFIUL ET AL: "Methods for artifact detection and removal from scalp EEG: A review", NEUROPHYSIOLOGIE CLINIQUE - CLINICAL NEUROPHYSIOLOGY, vol. 46, no. 4, pages 287-305, comprises a review of existing methods for detecting artifacts and removal of those artifacts from a scalp EEG.

Any discussion of documents, acts, materials, devices, articles or the like which has been included in the present specification is solely for the purpose of providing a context for the present invention. It is not to be taken as an admission that any or all of these matters form part of the prior art base or were common general knowledge in the field relevant to the present invention as it existed before the priority date of each claim of this application.

Throughout this specification the word "comprise", or variations such as "comprises" or "comprising", will be understood to imply the inclusion of a stated element, integer or step, or group of elements, integers or steps, but not the exclusion of any other element, integer or step, or group of elements, integers or steps.

In this specification, a statement that an element may be "at least one of" a list of options is to be understood that the element may be any one of the listed options, or may be any combination of two or more of the listed options.

Summary of the Description

According to a first aspect the present disclosure provides a system for separating a compound action potential from an artefact in a neural recording, the system comprising:

a memory storing a set of basis functions comprising at least one compound action potential basis function and at least one artefact basis function;
an input for receiving a neural recording of electrical activity in neural tissue; and
a processor configured to decompose the neural recording by determining at least one of a compound action potential and an artefact using the set of basis functions, and further configured to output an estimate of at least one of a compound action potential and an artefact.

According to a further aspect the present disclosure provides a method for separating a compound action potential from an artefact in a neural recording, the method comprising:

accessing a memory containing a set of basis functions comprising at least one compound action potential basis function and at least one artefact basis function;
receiving a neural recording of electrical activity in neural tissue; and
decomposing the neural recording by determining at least one of a compound action potential and an artefact from the set of basis functions, and
outputting an estimate of at least one of a compound action potential and an artefact.

According to an example there is provided computer software for carrying out the method of the second aspect.

According to another example there is provided a computer program product comprising computer program code means to make a computer execute a procedure for separating a compound action potential from an artefact in a neural recording, the computer program product comprising computer program code means for carrying out the method of the second aspect.

According to a further example there is provided a non-transitory computer readable medium for separating a compound action potential from an artefact in a neural recording, comprising instructions which, when executed by one or more processors, causes performance of the method of the second aspect.

The electrical activity in neural tissue may comprise an evoked compound action potential, evoked by an electrical stimulus applied to the neural tissue. The artefact basis functions may be matched to electrical artefact known to be caused by one or more such electrical stimuli.

In some embodiments of the disclosure, respective estimates of more than one underlying signal may be output, the underlying signals including the compound action potential signal component of the neural recording and the artefact component of the neural recording. In some embodiments the underlying signals may further include one or more of background neuronal activity, such as neuronal activity which is not evoked by the electrical neurostimulation, and/or an evoked late response as may result from evoked myoelectric activity.

In some embodiments of the disclosure, the or each estimate of the underlying signal(s) may be a deterministic estimate.

In some embodiments of the disclosure, both an evoked compound action potential (ECAP) and an artefact are simultaneously estimated. In such embodiments, the estimated ECAP and estimated artefact may be balanced to best represent the observed neural recording.

In some embodiments, an estimate of a noiseless ECAP may be output. In some embodiments, signal properties may be imposed upon some or all of the output estimates. A signal property of zero DC may be imposed upon the ECAP estimate, in some embodiments.

In some embodiments, a computational process for separating the compound action potential from the artefact in the neural recording may be computationally efficient, such as to order O(n). In some embodiments, a computational process for separating the compound action potential from the artefact in the neural recording may be executed in a deterministic time. In some embodiments, a computational process for separating the compound action potential from the artefact in the neural recording may be implemented in firmware of an implanted device.

In some embodiments, each underlying signal is represented as a linear combination of basis functions. In such embodiments, the basis functions may be derived in advance, such as being empirically derived and/or being derived from models of underlying signals. In such embodiments, the basis functions may be constant, or may be periodically updated such as by way of firmware upgrades.

In some embodiments, the ECAP may be modelled in the basis functions by a function matched to an expected noiseless ECAP morphology. For example the ECAP may be modelled in the basis functions by a piecewise function composed of one period of a sine wave followed by an exponential function such that the derivative is continuous at their boundary. The ECAP may be modelled in the basis functions by a function which is fitted to simulated ECAP models. The ECAP may be modelled in the basis functions by a parameterised function having parameters which allow time stretching, time shifting and/or DC offset. Such parameterisation may be constrained in order to maintain computational performance, minimise the impact of broadband noise and limit interference from artefact, while encompassing all expected real world ECAPs. The ECAP may be modelled in the basis functions by two or more basis functions, including for example a first basis function optimised for single ended neural recordings and a second basis function optimised for differential neural recordings. For example, the second basis function may be formed as a difference between two parametric ECAP basis functions, to reflect the nature of a differential recording as being the difference between the ECAP signals observed at each respective recording electrode.

In some embodiments, artefact may be modelled in the basis functions by three basis functions, comprising a DC basis function, a linear basis function, and an exponential basis function. The exponential basis function may for example be of the form $\phi_3(t) = e^{(-at)}$, where $a$ is a constant modelled or empirically matched to a relaxation component of artefact. For example, $a$ may be determined empirically from a library of human neurostimulation artefact recordings relevant to a device and stimulation paradigm in use. In one embodiment $a$ is in the range 10,000 - 70,000, more preferably in the range 30,000 - 50,000, more preferably in the range 35,000 - 40,000, and for example in one embodiment $a = 38,348$.

In alternative such embodiments, the exponential basis function may be substituted for a fractional pole scrubber.

Some preferred embodiments of the disclosure, further provide for detection of recordings in which only artefact exists, without any ECAP. In such embodiments, the signal is modelled using an Artefact-only basis function, and is also modelled using a combined ECAP and Artefact basis function. A set of signal features is derived from the estimates produced by both models and combined with signal features from the recorded signal. A series of signals known to contain both ECAP and Artefact or just Artefact were analysed by the present embodiment and the derived set of features saved. Machine learning was used to train a classifier with categories: 'ECAP' or 'no ECAP'. After sufficient training the resulting classifier is able to automatically judge the presence of ECAP in a signal.

According to a further example there is provided a method for removing artefact from a neural recording, the method comprising:

receiving a neural recording of electrical activity in neural tissue;
fitting a fractional pole model of artefact to the neural recording; and
removing the fitted fractional pole model from the neural recording.

In some embodiments of the disclosure the electrical stimulus applied to evoke the neural response may comprise a multipolar stimulus applied from three or more stimulus electrodes, the stimulus configured to evoke a neural response from only one phase of the stimulus and at only one site. For example, the multipolar stimulus may be configured in accordance with the teachings of International Patent Application No. PCT/AU2019/051151 by the present Applicant, the contents of which are incorporated herein by reference.

In some embodiments of the invention, the neural recording is obtained by an implanted spinal cord stimulator. Alternatively the neural recording may be obtained by an alternative neurostimulation device.

References herein to estimation or determination are to be understood as referring to an automated process carried out on data by a processor operating to execute a predefined estimation or determination procedure. The approaches presented herein may be implemented in hardware (e.g., using application specific integrated circuits (ASICs)), or in software (e.g., using instructions tangibly stored on computer-readable media for causing a data processing system to perform the steps described above), or in a combination of hardware and software. The invention can also be embodied as computer-readable code on a computer-readable medium. The computer-readable medium can include any data storage device that can store data which can thereafter be read by a computer system. Examples of the computer readable medium include read-only memory ("ROM"), random-access memory ("RAM"), CD-ROMs, DVDs, magnetic tape, optical data storage device, flash storage devices, or any other suitable storage devices. The computer-readable medium can also be distributed over network coupled computer systems so that the computer readable code is stored and executed in a distributed fashion.

Brief Description of the Drawings

An example of the invention will now be described with reference to the accompanying drawings, in which:

Figure 1 schematically illustrates an implanted spinal cord stimulator;
Figure 2 is a block diagram of the implanted neurostimulator;
Figure 3 is a schematic illustrating interaction of the implanted stimulator with a nerve;
Figure 4 illustrates a scrubbing process;
Figure 5 is a signal flow diagram;
Figure 6 illustrates ECAP and artefact basis functions, and their product;
Figure 7 illustrates the complete algorithm of one embodiment;
Figure 8 is an alternative illustration of a system for stimulating tissue and recording an ECAP;
Figure 9 illustrates the stimulus waveforms seen on recording electrodes;
Figure 10 illustrates a simplification of Figure 9;
Figure 11 illustrates separating resistive and capacitive components of input impedance; and
Figure 12 is a further simplification of the circuit under analysis.

Description of the Preferred Embodiments

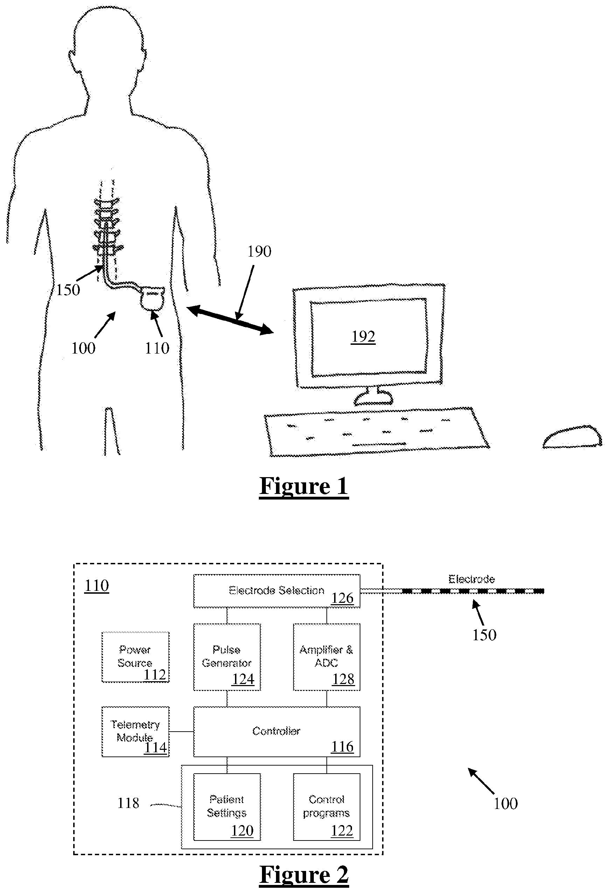

Figure 1 schematically illustrates an implanted spinal cord stimulator 100. Stimulator 100 comprises an electronics module 110 implanted at a suitable location in the patient's lower abdominal area or posterior superior gluteal region, and an electrode assembly 150 implanted within the epidural space and connected to the module 110 by a suitable lead. Numerous aspects of operation of implanted neural device 100 are reconfigurable by an external control device 192. Moreover, implanted neural device 100 serves a data gathering role, with gathered data being communicated to external device 192.

Figure 2 is a block diagram of the implanted neurostimulator 100. Module 110 contains a battery 112 and a telemetry module 114. In embodiments of the present invention, any suitable type of transcutaneous communication 190, such as infrared (IR), electromagnetic, capacitive and inductive transfer, may be used by telemetry module 114 to transfer power and/or data between an external device 192 and the electronics module 110.

Module controller 116 has an associated memory 118 storing patient settings 120, control programs 122 and the like. Controller 116 controls a pulse generator 124 to generate stimuli in the form of current pulses in accordance with the patient settings 120 and control programs 122. Electrode selection module 126 switches the generated pulses to the appropriate electrode(s) of electrode array 150, for delivery of the current pulse to the tissue surrounding the selected electrode(s). Measurement circuitry 128 is configured to capture measurements of neural responses sensed at sense electrode(s) of the electrode array as selected by electrode selection module 126.

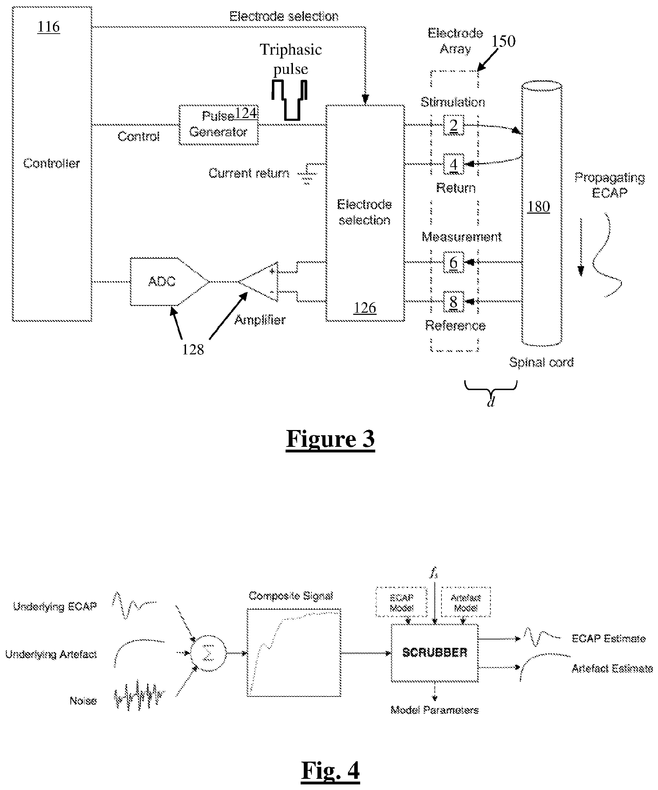

Figure 3 is a schematic illustrating interaction of the implanted stimulator 100 with a nerve 180, in this case the spinal cord however alternative embodiments may be positioned adjacent any desired neural tissue including a peripheral nerve, visceral nerve, parasympathetic nerve or a brain structure. Electrode selection module 126 selects a stimulation electrode 2 of electrode array 150 to deliver a triphasic electrical current pulse to surrounding tissue including nerve 180, although other embodiments may additionally or alternatively deliver a biphasic tripolar stimulus. Electrode selection module 126 also selects a return electrode 4 of the array 150 for stimulus current recovery to maintain a zero net charge transfer.

Delivery of an appropriate stimulus to the nerve 180 evokes a neural response comprising a compound action potential which will propagate along the nerve 180 as illustrated, for therapeutic purposes which in the case of a spinal cord stimulator for chronic pain might be to create paraesthesia at a desired location. To this end the stimulus electrodes are used to deliver stimuli at 30 Hz. To fit the device, a clinician applies stimuli which produce a sensation that is experienced by the user as a paraesthesia. When the paraesthesia is in a location and of a size which is congruent with the area of the user's body affected by pain, the clinician nominates that configuration for ongoing use.

The device 100 is further configured to sense the existence and electrical profile of compound action potentials (CAPs) propagating along nerve 180, whether such CAPs are evoked by the stimulus from electrodes 2 and 4, or otherwise evoked. To this end, any electrodes of the array 150 may be selected by the electrode selection module 126 to serve as measurement electrode 6 and measurement reference electrode 8. The stimulator case may also be used as a measurement or reference electrode, or a stimulation electrode. Signals sensed by the measurement electrodes 6 and 8 are passed to measurement circuitry 128, which for example may operate in accordance with the teachings of International Patent Application Publication No. WO2012155183 by the present applicant, the content of which is incorporated herein by reference. The present invention recognises that in circumstances such as shown in Figure 3 where the recording electrodes are close to the site of stimulation, stimulus artefact presents a significant obstacle to obtaining accurate recordings of compound action potentials, but that reliable accurate CAP recordings are a key enabler for a range of neuromodulation techniques.

The present invention provides a method for separating composite signals when signal components belong to a closed space of signals that may be represented by distinct basis sets. In neuromodulation this is used to separate the 'ECAP part' and the 'artefact part' of the recorded signals.

A composite signal is a signal that is constructed by the sum of other signals, which will be referred to here as the underlying signals. The basis element signal separation approach of the present invention estimates the underlying signals of the composite signal given only the composite signal, and without knowledge of the exact underlying signals. The present embodiment provides a blind signal separation algorithm which is able to assume some knowledge about the underlying signals. Namely, the present embodiment recognises that it can be assumed that each underlying signal may be represented by a set of basis functions. Unlike blind signal separation algorithms with multiple inputs and one output, the present invention produces a deterministic estimate of the underlying signals by leveraging this assumption.

In the field of neurostimulation, a mixed signal may be a combination of an ECAP and stimulus artefact. In some instances, there will be a need to decompose the signals and analyse the components. Analysing the individual components may reveal characteristics of the signal components which may be used in numerous advantageous ways. In some cases, analysing the components of the mixed signals may reveal errors in the system. Further, there may be situations where the mixed or composite signal has a dominant, but superfluous, component masking an essential component. In such cases, the mixed signal must be decomposed into its components, eliminate the superfluous component, and analyse the essential component and the characteristics thereof.

The present invention decomposes a mixed signal by determining at least one of the plurality of signals constituting the composite signal from a set of basis functions. The invention separates composite signals into their underlying components by modelling each underlying component with a basis. This invention may be applied in neuromodulation in the separation of ECAP waveforms from artefact waveforms (as well as noise) given a signal recording which is a mixture of these signals. This yields more robust feature extraction from the ECAP, including the ECAP magnitude which is a feature used during operation of the closed loop control system of Figs 1-3. Additional features such as ECAP peak positions may also be measured more robustly, which may be of scientific benefit and/or operational benefit. The present embodiment estimates both artefact and ECAP simultaneously, where ECAP and artefact signal contributions are balanced to 'best' represent the recorded signal. The present embodiment produces a noiseless ECAP estimate and subject to the definition of the ECAP basis set, can impose certain signal properties (e.g. a baseline of 0V). Further, the present embodiment is efficient (O(n)) and runs in a deterministic time (unlike non-deterministic methods), which means that it may be potentially integrated into firmware, giving improved, real-time ECAP magnitude estimates without the need of a human tuned filter.

A scrubber is an algorithm that estimates the ECAP and Artefact components of some composite signal. A composite signal is defined as a signal composed of the sum of multiple distinct elements. In the context of ECAP measurement the components of a composite measurement are the artefact, the neurophysiological response to the stimulus (the ECAP), and everything else. The primary goal of a scrubber is to isolate the ECAP. However, artefact estimation is usually a by-product of this task and is useful in and of itself as insights into the mechanism of artefact will help us to minimise it in future designs. What is left over consists of electronic noise and neurophysiological noise independent of stimulation. **Fig.4** illustrates this process.

The present embodiment adopts the following process. Each underlying signal is represented as a linear combination of basis functions. Consider a composite signal with two underlying signals.

$$\sigma(x) = f(x) + g(x) \approx \sum_k \alpha_k \, \phi_k(x) + \sum_j \beta_j \, \varphi_j(x)$$

Basis functions are derived empirically based on experience and alternate models of underlying signals. For the purposes of explanation, consider them to be constant. Computing the pairwise inner products of basis functions and the inner product between each basis function and the composite signal, one may write down a set of linear equations that may be solved with matrix inversion to obtain the sets of coefficients alpha and beta. Given the alpha coefficients, one may then write down the basis representation of *f(x)*, thus estimating *f(x)*. Similarly, one may estimate *g(x)* given the beta coefficients. This method is not limited to composite signals containing two components, but the problem it is applied to in the described neuromodulation field has just two components.

The basis element signal separation approach of the present embodiment is a mathematical tool for deconstructing composite signals. Consider a signal containing an ECAP component $f(t)$ and an Artefact component $g(t)$. The signal that we measure in a patient $\sigma(t)$ may therefore be expressed as:

$$\sigma(t) = f(t) + g(t) + e(t)$$

where $e(t)$ is some noise. Closed loop stimulation works because the ECAP component of a given signal has a regular shape which resembles two periods of a dampened oscillation. In a similar vein, closed loop stimulation would not work if the artefact component of the signal did not have a regular shape. In order to measure ECAP amplitude we filter out most of the artefact using the detector, which assumes that the artefact has a regular exponential-like shape.

The present embodiment operates on the assumption that ECAP and artefact signal components belong to distinct families of functions. That is, ECAPs are always short oscillatory events, whilst artefacts are exponential-looking signals. For each distinct family of functions we can predefine a basis to represent it. For suitable basis functions, the basis coefficients can be calculated and the ECAP and artefact basis expansions can each be isolated. The ECAP basis expansion then provides us an estimate of the ECAP component, free from artefact.

The calculation of basis coefficients balances the contributions of each of the basis functions in such a way that the overall signal is approximated as best as possible. In other words, the estimated ECAP and Artefact contributions are balanced so as to best model the signal that has been recorded. In order to do achieve better performance, the present embodiment assumes that all ECAPs belong to a certain family of functions and that ECAP shapes outside of this family do not exist. At the time of writing, ECAPs with late responses such as those set forth in WO2015070281 are outside the family of ECAP functions used by the present embodiment and therefore cannot be estimated properly. Therefore, other Scrubbers may be more appropriate to use when working with signals not adequately modelled by the ECAP basis in use at the time. Alternative embodiments of the present invention may incorporate basis functions which include ECAPs with late responses within the family of ECAP basis functions, to provide for late response estimation.

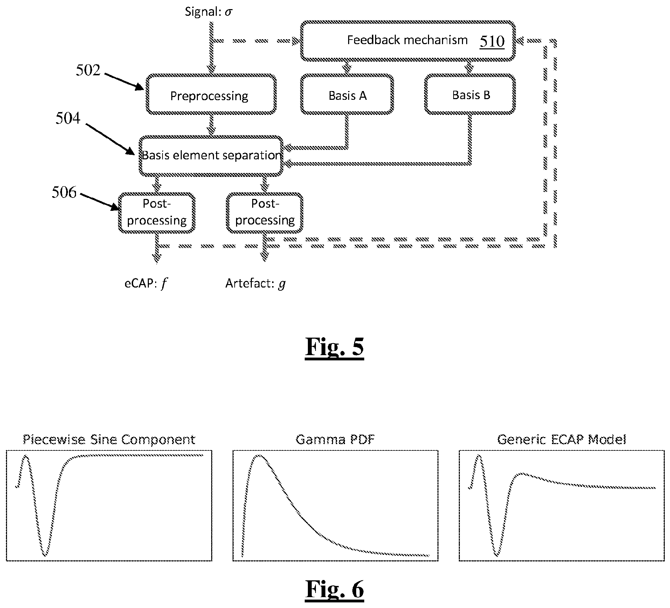

The method described above forms the block 504 in the signal flow diagram of **Fig. 5.** Pre processing 502 and post processing 506 are used, in some embodiments, to improve signal estimates. For example, pre-processing 502 can be used to reduce high frequency noise in the signal. The feedback mechanism 510 however is used to improve the construction of basis sets. A crude 'first guess' basis may be used to approximate the signal and the estimates that are produced can be used to refine the basis set on subsequent passes. For example, the first pass might guess an ECAP basis in order to get a good estimate of the artefact. Subtracting the artefact from the signal and using signal correlation methods can be used to refine the choice of ECAP basis. Rerunning the algorithm with the improved basis will yield better estimates of both the ECAP and the artefact.

Artefact is modelled by the present embodiment using three basis functions:

$$\phi_1(t) = 1 \quad , \quad \phi_2(t) = t \quad , \quad \phi_3(t) = \exp\left(-\frac{16.384 \times 10^3}{7.0}t\right)$$

The unit basis function $\phi_1$ captures the DC content of the measured signal. The linear basis function $\phi_2$ captures the component of Artefact due to amplifier drift. The exponential basis function $\phi_3$ captures the chemical charge relaxation component of the Artefact. The decay constant of the exponential component can be any suitable variable and the value above was determined empirically based on model performance against a library of human Artefact recordings. Different devices may present different artefact and/or ECAP outcomes and may consequently require different constants, which can be similarly empirically obtained.

Once the algorithm of the present embodiment is applied, the Artefact component of the signal is represented by:

$$A(t) = \alpha\phi_1(t) + \beta\phi_2(t) + \gamma\phi_3(t)$$

This model, while simple, has been applied to many thousands of representative human patient neural recordings. In combination with the ECAP basis functions, the combined model accurately estimates the recorded signal.

Unusual neurological Artefact such as background neuronal activity or late response are not modelled in the present embodiment, but may be incorporated in accordance with alternative embodiments of the invention. Estimates obtained from the approach of the present embodiment will remove such features and therefore the outcome cannot be relied upon in the measurement of non-ECAP neurological features, at least in this embodiment.

An ECAP basis function is defined using the product of a Gamma probability density function, with parameters k = 1.7 and $\theta$ = 0.60,

$$\varphi(t) = (ft)^{k-1} \cdot e^{-ft/\theta}$$

This is a piecewise function composed of one period of a sine wave followed by an exponential function such that the derivative is continuous at their boundary:

$$\phi(t) = \begin{cases} 0 & t < \arcsin(C)/2\pi f \\ \sin(2\pi ft) - C & \arcsin(C)/2\pi f < t < 1/f \\ 1 - C - e^{-2\pi f(t - 1/f)} & t > 1/f \end{cases}$$

where C = 0.37. The two components and their product are represented in **Figure 6.** There is only one morphology parameter in this FPAP model; the frequency of the sinusoidal component: f . As can be seen above, the timescale of the Gamma PDF is scaled accordingly. This model was arrived at through the hand fitting of elementary functions to simulated ECAP models.

By scaling the time axis by v and applying an offset t0: $v(t - t_0)$, we can stretch and shift an ECAP basis function in time. Let such a stretched and scaled ECAP be called a parametric ECAP basis function: $\varphi_{v,t0}(t)$.

There are two distinct ECAP models, one for singled ended measurements (in which the recording is made from a single electrode relative to an indifferent reference such as device ground) and another for differential measurements (in which the recording is made from two recording electrodes both exposed to the neural signal). The single ended ECAP basis consists of one parametric ECAP basis function and the ECAP E is represented by:

$$E(t) = \kappa \varphi_{\nu,t_0}(t)$$

The differential ECAP basis is formed by the difference of two parametric ECAP basis functions giving the following ECAP model

$$E(t) = \kappa_+ \varphi_{\nu_+,t_{0+}}(t) - \kappa_- \varphi_{\nu_-,t_{0-}}(t)$$

In either model, the time stretch (corresponding to the ECAP oscillation frequency) and the time offset are chosen such that $\kappa$ or $\kappa_+$ is positive and $\kappa_-$ is negative. A sweep of ECAP frequencies and offsets are tested by the present embodiment to ensure this condition holds. The frequency and offset selected to model the ECAP component of a recorded signal are chosen such that the fit to the recording using both ECAP and Artefact models is as good as possible.

It should be noted that the single ended ECAP model assumes fixed ratios between peak heights and peak times. Neurophysiological parameters such as width at half height or the $n_1 : p_2$ ratio are entirely determined by the temporal stretch v applied to the parametric basis function.

As with Artefact, this assumption has been validated by fitting parametric basis functions to real-world single ended measurements.

In the case of the differential model, such neurophysiological parameters are able to vary independently of $v_+$ and $v_-$ and additionally depend on the composition of the ECAP estimate. That is, $\kappa_+$ and $\kappa_-$ provide additional degrees of freedom. Although relative neurophysiological parameters are able to vary they have restricted freedom compared to more free-form ECAP models. As with the single ended ECAP assumption, this model constraint has been validated by fitting the differential ECAP basis to real-world differential measurements.

The range of parametric ECAP frequencies is limited to a linearly spaced set of frequencies between 500Hz and 2kHz. The upper limit of 2kHz was chosen to minimise the interference of broad spectrum (up to 8kHz) noise on the parameter selection procedure. The lower limit of 500Hz was chosen to limit the interference of the Artefact on the parameter selection procedure. A slow enough parametric ECAP will closely resemble Artefact in a confined window of time. The range of offsets that are tested was chosen to be sufficiently wide to model real-world ECAPs, but reasonably constrained to maintain computational performance.

Up until this point, we have assumed that each recorded signal contains an ECAP. However, in practice this is never the case for signals that are sub-threshold. Including ECAP basis functions in the model for a sub-threshold signal poses a problem, as an ECAP would be fitted to the noise in the signal and the estimate would be meaningless. Additionally, the Artefact component of the signal would be misrepresented as ECAP and Artefact features are balanced in a combined model.

It is therefore desirable to include a mechanism that detects the presence of ECAP in a signal so that the ECAP basis may only be included in the overall model when an underlying ECAP is authentic. The present embodiment incorporates such a mechanism. The signal is modelled using an Artefact only basis and a combined ECAP and Artefact basis. A set of signal features is derived from the estimates produced by both models and combined with signal features from the recorded signal. A series of signals known to contain both ECAP and Artefact or just Artefact were analysed by the present embodiment and the derived set of features saved. Machine learning is used to train a classifier with categories: 'ECAP' or 'no ECAP' . After sufficient training the resulting classifier is able to automatically judge the presence of ECAP in a signal. The present embodiment is rated to detect ECAP in signals containing ECAP with an accuracy of 85% and to reject ECAP in signals containing only Artefact with an accuracy of 95%.

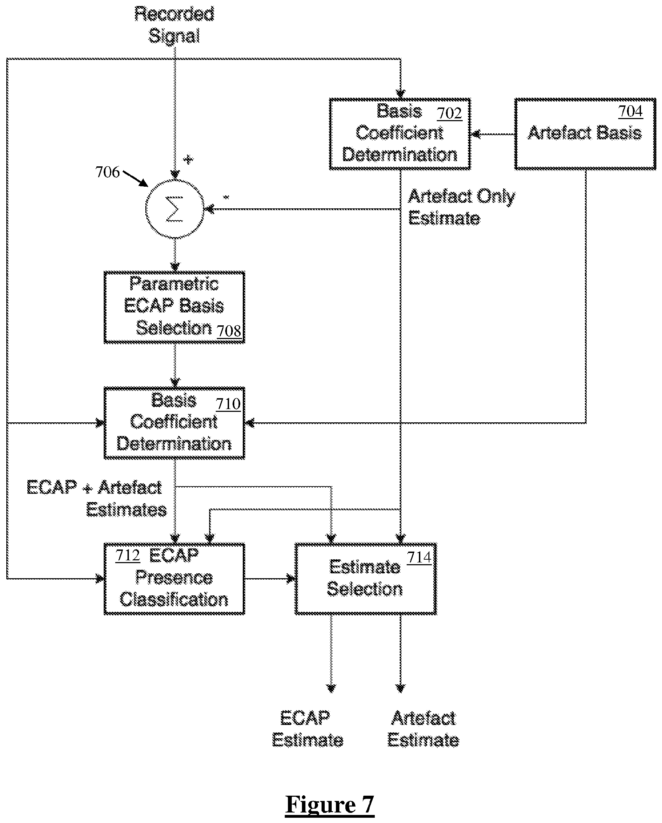

Combining these concepts together, we arrive at the complete algorithm of the present embodiment as depicted in Fig. 7.

The recorded signal is first modelled at 702 using an Artefact only basis 704, under the assumption that the recording contains no ECAP. Regardless of ECAP presence this will provide an estimate of the Artefact via the basis coefficients. If an ECAP is present this estimate may be refined by including an ECAP basis as well. The initial Artefact estimate is subtracted off the recorded signal at 706 to help better determine the parametric ECAP basis. The estimated Artefact and derived features from 702 are also passed to the 'ECAP Presence Classification' (or ECAP detector) block 712 for later use.

Once the parameters for the Parametric ECAP Basis are determined at 708, the coefficients of the ECAP and Artefact basis in conjunction are then determined at 710. Resulting estimates and feature sets passed to the ECAP detector 712.

The ECAP detector 712 now has everything it needs in order to classify the presence of ECAP in the recorded signal. Based upon its decision, the estimate selection block 714 returns either the ECAP and Artefact estimates, or the Artefact only estimate.

The method steps are as below:

a. Capturing/ recording a composite signal, wherein the composite signal has two or more additive components
b. Selecting a first basis set, corresponding to the first signal component, from a pool of basis sets. Selecting a second basis set, corresponding to the second signal component, from a distinct pool of basis sets.
c. Determining a first component and the second component of the composite function based on the bases functions. Determining an estimate for the first component as a linear expansion of the first basis set, and an estimate for the second component as a linear expansion of the second basis set.
d. Iteratively improving the basis sets using the estimated components from the previous iteration.

The following explanations delve into the mathematics behind the present embodiment. Coefficient Determination is as follows. Let $\sigma(t)$ be the signal we record, and $f(t)$ and $g(t)$ the underlying ECAP and Artefact components respectively. The problem we are attempting to solve is to find estimates for $f(t)$ and $g(t)$, which we do not know, using the recorded signal $\sigma(t)$, which we do know. For simplicity, we assume there is no noise in the signal. Therefore,

$$\sigma(t) = f(t) + g(t)$$

Now suppose that $f(t)$ may be represented using a finite set of basis functions $\{\varphi_k(t): k \in \{1, 2, ... n\}\}$. Similarly, suppose that $g(t)$ may be represented using a finite set of basis functions $\{\phi_j(t): j \in \{1, 2, ... m\}\}$ all distinct from the set used to represent f(t). Then $f(t)$ and $g(t)$ may be expanded over their respective bases,

$$(2) \qquad f(t) = \sum_{k=1}^{n} a_k \varphi_k(t)$$

$$(3) \qquad g(t) = \sum_{j=1}^{m} b_j \phi_j(t)$$

Then by simple substitution:

$$\sigma(t) = \sum_{k=1}^{n} a_k \varphi_k(t) + \sum_{j=1}^{m} b_j \phi_j(t)$$

At this stage of the problem, the basis sets are known but the coefficients for the specific signal $\sigma(t)$ are not. With the coefficients we may recover estimates *for f(z)* and *g(t).* We will recover them now.

Consider the following functional inner product for any basis function of $f$: $\varphi_i(t)$ and by the linearity of inner products we have:

$$(4) \qquad \langle \sigma(t), \varphi_i(t) \rangle = \sum_{k=1}^{n} a_k \langle \varphi_k(t), \varphi_i(t) \rangle + \sum_{j=1}^{m} b_j \langle \phi_j(t), \varphi_i(t) \rangle$$

Similarly, consider the functional inner product for any basis function of g: $\phi_l(t)$

$$(5) \qquad \langle \sigma(t), \phi_l(t) \rangle = \sum_{k=1}^{n} a_k \langle \varphi_k(t), \phi_l(t) \rangle + \sum_{j=1}^{m} b_j \langle \phi_j(t), \phi_l(t) \rangle$$

Equations (4) and (5) provide us with a system of $n + m$ linear equations with $n + m$ unknowns (the coefficients $a_k$ and $b_j$). Thus, determining the coefficients is a matter of solving a linear equation:

$$Hv=b$$

where

$$H = \begin{pmatrix} \langle \varphi_1, \varphi_1 \rangle & \langle \varphi_1, \varphi_2 \rangle & \cdots \\ \langle \varphi_2, \varphi_1 \rangle & \ddots & \vdots \\ \cdots & \cdots & \langle \phi_m, \phi_m \rangle \end{pmatrix} \quad, \quad b = \begin{pmatrix} \langle \sigma, \varphi_1 \rangle \\ \vdots \\ \langle \sigma, \phi_m \rangle \end{pmatrix}$$

Thus the coefficients may be solved via $H^{-1}b$. The matrix $H$ is invertible if and only if none of the basis functions from the ECAP basis belong to the span of the Artefact basis and vice versa, and basis functions with ECAP and Artefact bases are distinct. Basis functions should be scaled to unit power so that comparatively large or small inner products do not introduce computational error during the inversion of $H$.

In practice there is noise in the signal which is not modelled by either basis. However, introduced errors will be minor since the inner product of an independent noise source and any signal is zero for an inner product taken over an infinite time interval. Limiting the inner product to a finite number of samples when calculating $b$ will propagate some error, however, this error is not significant.

ECAP Parameter Determination is as follows. The parametric ECAP basis is determined using the recorded signal with the initial Artefact removed and any residual baseline subtracted. Let this signal be called the 'refined recording'. A correlation mesh is determined by sweeping a range of basis ECAP frequencies and offsets and taking the dot product between the refined recording and each parametric basis function.

For single ended and differential mode, the present embodiment samples 16 linearly spaced frequencies between 800Hz and 2kHz and offsets from -7 samples to -1 samples inclusive. This range of frequencies and offsets was found to work well against test signals observed in human subjects but these ranges may be extended. Extending them too far will allow the parametric ECAP to lock onto noise or the Artefact so do so with caution. The highest positive stationary point of the correlation mesh determines the parameters of the first ECAP basis element. If the measurement is single ended, then this is the only ECAP basis element.

In the case of a differential ECAP measurement, a new correlation mesh is calculated, sampling 16 linearly spaced frequencies between 500Hz and the frequency of the previously determined basis element. It is assumed that the reference is always further away from the stimulus than the recording electrode. This allows us to exploit human neurophysiology since ECAP frequency monotonically decreases with recording distance. In a similar vein, offsets are tested between the previous ECAP basis offset and 12 samples. Again these ranges were empirically chosen to work well with good signals from humans. Instead of using the highest positive stationary point of the correlation mesh, the most negative stationary point instead determines the parameters of the secondary basis function. If there are no negative stationary points, only the primary basis function is utilised.

The majority of blind signal separation algorithms assume that the underlying signals are statistically independent and use statistical signal processing techniques to estimate the underlying signals. The problem of ECAP and artefact estimation cannot be solved in this way because the underlying signals are fundamentally dependent on one another. Instead the present embodiment assumes that each underlying signal may be expressed as a linear combination of basis functions (a stronger assumption) limiting its application to processes where there is already some knowledge of the underlying signals before they are recorded in the form of a composite signal.

The Artefact Model lists the basis functions used to model the Artefact present in our hardware/recordings. The FPAP model is a singular basis function used in the total ECAP basis set. In practice we use one FPAP for single ended measurements and two FPAPs for differential measurements to take care of the reference electrode effect arising with differential measurements taken between two recording electrodes.

Alternative embodiments are further provided. In this embodiment the process of Fig. 4 is instead implemented as follows.

An Artefact Estimation Scrubber is a Scrubber that attempts to estimate only the Artefact component of the signal g(t) and derives an ECAP estimate using σ(t) - g(t). Exponential Scrubbers model the Artefact as the sum of exponential functions. There are three such models envisaged here:

Table 1: The Exponential Scrubber Artefact models

|  | Exponential | Time domain representation |
|---|---|---|
| Single | $g(t) = a \exp(-bt) + h$ | |
| Double | $g(t) = a \exp(-bt) + c \exp(-dt) + h$ | |
| Triple | $g(t) = a \exp(-bt) + c \exp(-dt) + f \exp(-gt) + h$ | |

A non-linear optimisation is performed using the simplex hill-climbing Nelder Mead algorithm where the parameters a; b; c; d; e; f; g and h are all tuned to minimise the value of a cost function. The non-linear optimisation minimises the sum of the squares error between the estimated Artefact samples and the samples of the recorded signal. Mathematically, the cost function is defined as:

$$E(g, \sigma) = \sum_{i=0}^{n} (\sigma[i] - g[i])^2$$

Non-linear optimisations are non-deterministic algorithms, meaning that they do not terminate in a predictable or pre-determinable amount of time. That means that it is possible to provide such a scrubber with a signal that cannot be scrubbed in a reasonable time frame. Further, non-linear optimisations can become stuck in local minima, failing to find the true optimal solution. In practice, this Scrubber works well but it has limitations that should be known before putting it to general use. Nevertheless such embodiments do have uses in certain applications.

A further embodiment is a fractional pole Scrubber which works on the same principles as the exponential Scrubbers where a non-linear optimisation is used to determine parameters a; k; α and h of the following Artefact model:

$$g(t) = a \exp(-kt) \cdot t^{1.0-\alpha} + h$$

In more detail, it has been noted by the present inventors that electrode impedance variation is commonly observed clinically in humans, and that such electrode impedance variations can affect the behaviour of the electrode-tissue interface and give rise to artefact. Indeed, such variations may affect the constant phase element model of the electrode-tissue interface more than the resistive part of the impedance. The present embodiment thus recognises that artefact arising from such characteristics can best be dealt with by understanding the impact of a difference in electrode impedance between one electrode and the next. To this end, we consider a split electrode model of constant phase elements, as set forth in International Patent Application No. PCT/AU2019/051160 by the present Applicant, the contents of which are incorporated herein by reference.

The electrode-tissue is the interface between the aqueous, ion-rich environment of the human body and the charged metal lattice of an implanted electrode. In real terms, the aqueous ions display unique behaviours in response to rapid charging and discharging of the metal lattice, principally characterised by the rapid formation and diffusion of a bilayer of ions on the electrode surface. This is known as the ionic double layer and it has both capacitive and resistive characteristics. The aggregation and diffusion of ions from the metal surface is purely capacitive in an ideal system. However, a reversible modification of ionic species and exchange of electrodes at the metal surface is also known to occur with a voltage-dependent rate. As such we electrically characterise the electrode-tissue interface using a concept known as the Constant Phase Element (CPE), which is effectively a leaky capacitor. The CPE is formed when metal comes in contact with the ionic fluids in tissue. In a saline bath, the entire electrode surface contributes to conduction, though when implanted, material such as bone or encapsulation tissue in close proximity will reduce the contact's effective size and so increase the CPE component of the impedance. Impedances of individual electrodes along an implanted lead can vary by more than 2:1.

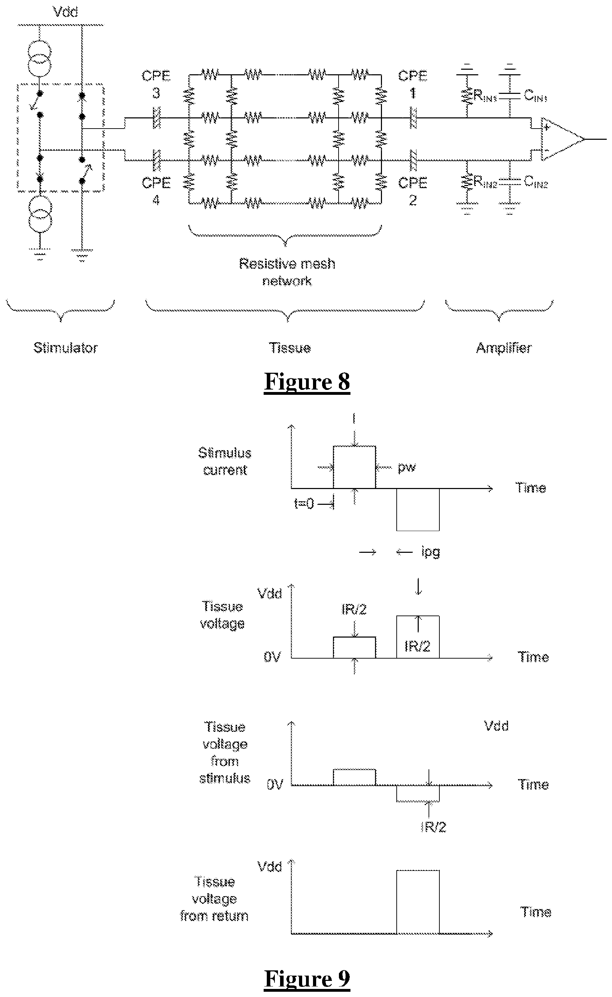

Figure 8 shows a system for stimulating tissue and recording an ECAP, similar to that of Figure 1. The tissue is shown as a resistor mesh to which the electronic components connect via constant-phase elements (CPEs). This representation is explained in detail in International Patent Application No. PCT/AU2019/051160. In this diagram, the system stimulation electrodes are represented by CPE3 and CPE4, and the recording electrodes by CPE1 and CPE2. On the electronics connections to CPE1, 2 etc will be referred to as electrode 1, electrode 2 etc.

The stimulator in this system can produce stimuli with one or more phases by operation of the switches and current sources. The current amplitudes and switch timing are controlled by the stimulator, and this is known to the prior to stimulation occurring. The operation of the switches connecting the tissue to Vdd or ground causes currents to flow through CPE1 and CPE2 and into the amplifier resistive and capacitive input impedances.

The method described in this disclosure is relevant to situations when the recording electrodes associated with CPE1 and CPE2 are sufficiently distant from the stimulation sites that that can be considered to be at the same potential, at least on the tissue connecting sided. That is to say, the current flow through the resistive mesh close to these electrodes is effectively zero. When the system is delivering an anodic stimulus to electrode 3, it connects electrode 3 to ground, and electrode 4 to the positive current source. The voltage on the recording electrodes 1 and 2 will be somewhere in between the voltages on electrodes 3 and 4, depending on the details of the mesh. For convenience, it will be assumed that this fraction is one half i.e. midway in between. If the impedance between the stimulating electrodes is R, then this voltage will then be IR/2.

When the system is delivering a cathodic stimulus to electrode 3, it connects electrode 3 to Vdd, and electrode 4 to the negative current source. Again, the tissue voltage is assumed to be midway between these. When the tissue is not being driven, the tissue can be connected to Vdd or ground, and in this case ground will be chosen though it is arbitrary. Choosing ground is the preferred method as this does not require maintaining a high-voltage power supply when not stimulating.

In the case of a biphasic stimulus, the waveform is as per the second panel of Figure 9, which illustrates the stimulus waveforms seen on recording electrodes. It will be observed that this waveform is the sum of the electrode to power supply voltage and the electrode to electrode voltage.

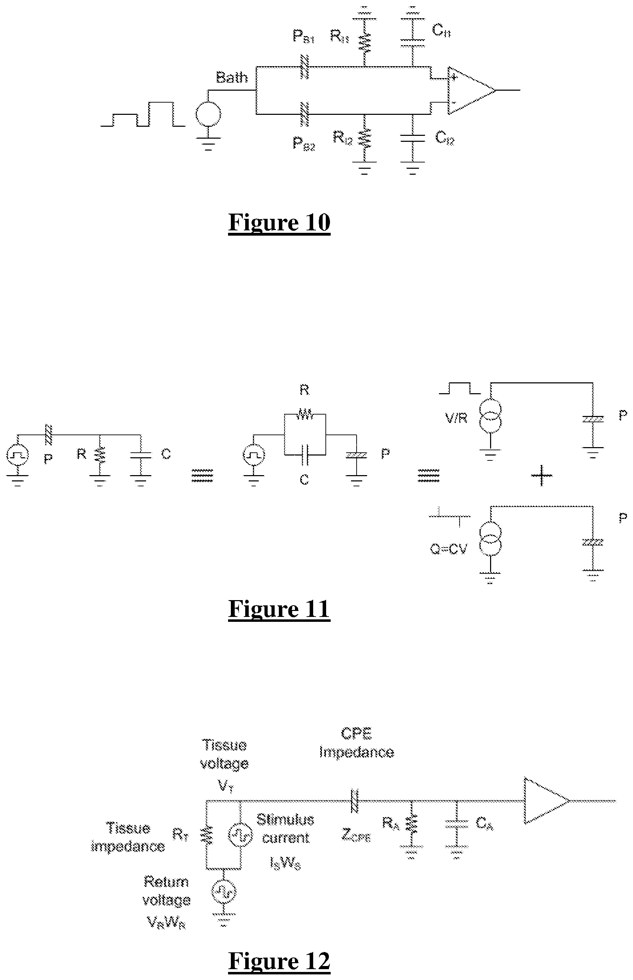

Since the voltages on electrode 2 and electrode 3 are the same, and the waveform is as described in Figure 9, then Figure 8 can be simplified to Figure 10. If we assume that by careful design and component control $R_{I1} = R_{I2}$ and $C_{I1} = C_{I2}$ then there will only be a non-zero voltage at the amplifier output if $P_{B1} \mathrel{!=} P_{B2}$. This would be expected in practice, because these electrodes are placed in tissue and is not homogenous, so it would be expected that these impedances will not be the same.

If we now calculate the impedance on each amplifier input calling $Z_i$ the impedance of the input circuit and $Z_c$ the impedance of the CPE, so as a simple voltage divider:

$$V_1 = \frac{Z_i}{Z_i + Z_{c1}} = \frac{1}{1 + {Z_{c1}}/{Z_1}}$$

Since $Z_i \gg Z_{c1}$ using Newton's approximation: $(1 + p)^q = 1 + pq$ if $pq \ll 1$ then we can write:

$$V_1 \cong 1 - {Z_{c1}}/{Z_i}$$

The voltage between the amplifier inputs is

$$V_1 - V_2 \cong \frac{1}{Z_i}(Z_{c2} - Z_{c1})$$

i.e. the voltage is proportional to the difference in the impedances. Since $Z_{c1}$ and $Z_{c2}$ are fractional poles, this impedance will also have the properties of a fractional pole.

Figure 11 illustrates separating resistive and capacitive components of input impedance. Because the current applied to the system is zero during recording, and again because $Z_i \gg Z_{c1}$ the transformation of Figure 11 breaks currents through the CPE into resistive and capacitive components. Since the waveforms are rectangular, the voltage pulses produce current pulses into the CPE due to the resistor, and the edges produce impulses through the capacitor.

The impulse response of a CPE is the inverse Laplace transform of a fractional pole and is given by the equation

$$i(t) = k\, t^{-\alpha}\, for\, t \geq 0$$

and Equation

$$i(t) = 0\, for\, t < 0$$

From this the step response can be simply calculated as

$$s(t) = \int_{t=0}^{t=\infty} k\, t^{-\alpha} dt = \frac{k}{1-\alpha} t^{1-\alpha} + C \qquad \text{Equation 1}$$

The step response can be extended to describe the pulse response for a pulse of width T as

$$p(t,T) = s(t) - s(t-T) \qquad \text{Equation 2}$$

This can equivalently be extended to more complex waveforms such as biphasic and triphasic pulses.

The waveforms of Figure 9 have two components: the biphasic stimulus pulses and the rectangular tissue-return pulse. The response of the CPE can be separated into the resistive (pulse) and capacitive (impulse) response from the amplifier input impedance terms. Thus the artefact generated at the amplifier output is the sum of the resistive and capacitive components and the biphasic and supply-return pulses.

Naming the pulses related to the biphasic pulses with "b", those related to the stimulus return pulses as "s", those from the rectangular resistive pulses as "r" and from the impulsive capacitor pulses as "c", these four terms are:

$$y(r,b) = k(r,b)\,[s(t) - s(t-pw) + s(t-pw-ipg) - s(t-2.pw \qquad \text{Equation 3}$$
$$- ipg)]$$

$$y(c,b) = k(c,b)\,[i(t) - i(t-pw) + i(t-pw-ipg) - i(t-2.pw \qquad \text{Equation 4}$$
$$- ipg)]$$

$$y(r,s) = k(r,s)\,[s(t-pw-ipg) - s(t-2.pw-ipg)] \qquad \text{Equation 5}$$

$$y(c,s) = k(r,s)\,[i(t-pw-ipg) - i(t-2.pw-ipg)] \qquad \text{Equation 6}$$

The total artefact

$$y = y(r,b) + y(c,b) + y(r,s) + y(c,s) \qquad \text{Equation 7}$$

Each of these terms has a constant of proportionality e.g. *k(r, b)*. These will depend on the difference in the impedance of the CPEs ($Z_{c2}$ - $Z_{c1}$), the (variable) amplitude of the stimulus and the fixed amplitude of the supply voltage, and the values of the resistors $R_i$ and the capacitors $C_i$.

However, the present embodiment recognises that these four terms could be used in a scrubber which finds the most likely match of a sum of these components and an ECAP.

If the amplifier has an input impedance that is purely capacitive, then *y(r,s)* = *y(r,b)*= 0. In this case the corresponding "k" values do not need to be found, leaving just two unknowns to be solved for.

The relationship between the capacitive and resistive impedance parts of a CPE is described in International Patent Application No. PCT/AU2019/051160. This can be used to allow $y(r, b)$ to be calculated from $y(c, b)$ reducing the number of variables that must be evaluated.

Finally, if the tissue impedance has been measured, the stimulus current and supply voltages are known, then the tissue voltage can be calculated and it can be used instead of the four equations described previously.

It has been observed that the term $\alpha$ varies with electrode geometry. Therefore the scrubbing process may also need to vary this parameter during its optimization.

An investigation into the real scrubbing performance of a mathematical basis for the origin of stimulation artefact was performed, using mixed ECAP and Artefact signals recorded in human SCS patients. The artefact basis described below was investigated for scrubbing performance by implementation as a modified version of the Basis Element Signal Separator described in the preceding.

As noted above we can describe the voltage response of a CPE with two component voltage signals which have the properties of a fractional pole.

Each of these time varying components represent one of the distinct capacitive and resistive behaviours of the amplifier input impedance. One component has a positive slope and is representative of the voltage produced by the resistive component of the amplifier input impedance. We term this the step component:

$$s(t) = k_s \frac{1}{1-\alpha} t^{1-\alpha}$$

The other has a negative slope and represents the interaction of the CPE and the capacitive component of the amplifier input impedance. We term this the impulse component:

$$i(t) = k_i t^{-\alpha}$$

The time constant $\alpha$ for the fractional poles is thought to be dependent on the geometry of the electrode-tissue interface. For this analysis we have assumed a constant value:

$$\alpha = 0.364$$

The scalar components for the step ($k_s$) and impulse ($k_i$) are assumed to be dependent on the amplitude of stimulation current delivered to the stimulating electrodes and the characteristics of tissue between stimulation and recording sites, for which we do not control here. Importantly, we assume that $k_s$ and $k_i$ remain constant for a given source (i.e. a stimulating and recording electrode set).

For a given biphasic or triphasic stimulation paradigm, the stimulation and return voltage waveforms will contain multiple steps. Each edge of a voltage step acts as a singularity at which we can define an independent set of step and impulse components. The number and timing of these edges/singularities can be exactly defined based on the stimulation waveform and depend on the following adjustable parameters of the system:

- Number of Phases (Biphasic or Triphasic).
- Polarity of the First Phase (Negative or Positive).
- Pulse Width
- Interphase Gap

The recorded artefact is the scaled sum of all of the time-offset step and impulse components generated by the stimulation and return voltage waveforms. We can define general equations for these time-offset stimulation and return step and impulse responses which use these known parameters of the stimulation waveform:

The artefact basis described above was also used to build an implementation of an artefact scrubber by modifying the Basis Element Signal Separator described in the preceding. As noted above, the Basis Element Signal Separator uses three basis functions to model the artefact:

- An exponential of fixed time constant
- A linear slope
- A DC offset

The present embodiment modifies this by replacing the exponential artefact basis with the fraction pole model described above. K values were fit to a representative artefact and then parsed to the Basis Element Signal Separator and remained fixed to scrub all the signals for a given activation plot.

Note that the Basis Element Signal Separator performs linear operations on the artefact and ECAP basis to optimise the fit to the underlying signal. That is, for this implementation the fractional pole artefact basis need not specify the magnitude and polarity of the artefact basis exactly, only its temporal and shape characteristics, as the Basis Element Signal Separator performs additional scaling as part of fitting.

Results from fitting bounded k values using a non-linear method showed that the model artefact was able to match the temporal and shape characteristics of the representative artefact in all cases but was missing some linear characteristics of the recorded signal

. Parsing this model artefact to the Basis Element Signal Separator, the resulting fit to the artefact was of a consistent high quality with minimal error, indicating that only linear characteristics were missing from the model

We tested the performance of our modified Basis Element Signal Separator implementation as an artefact scrubber when working with mixed ECAP and artefact signals. For each input activation plot, the artefact basis was parameterised using a representative artefact, as described above. Also signals from the activation plot were then scrubbed using the parameterised model and the results were visualised. The scrubber demonstrates effective removal of the artefact and residual ECAP signal is flat and clean. Additionally, the scrubbed activation plot has none of the subthreshold offset or slope which is present in the original.

The circuit we are considering can be distilled to Figure 12, which may assist to understand the relationships between the variables.

There are two stimulus waveforms:

1. The voltage formed by the current source output multiplied by the tissue impedance. This varies with tissue impedance and with stimulus current; the first is unique to the electrodes and the latter is known from the programming system (one unknown).
2. The return voltage (VddHV). This is known from the programming system.

Both the waveforms have an amplitude part and a shape part. The shape part is known from the programming system. The tissue voltage is the sum of the return voltage and the stimulus voltage:

$$V_T = V_R rs + R_T I_S css$$

The CPE stores the charge that flows into the amplifier input impedance, and the redistribution of this charge leads to the artefact.

The capacitance provides the path that creates an impulsive response from the stimulus waveforms, it differentiates the tissue voltage to provide the impulsive components.

This boils down to the following equation which has a small number of variables and if implemented in a scrubber finds the physical values of the parasitic components $Z_{CPE}$, $R_A$ and $C_A$.

$$I_A = Z_{CPE}\left(\frac{1}{R_A}\left(V_R rs + R_T I_S css\right) + C_A \frac{d}{dt}\left(V_R rs + R_T I_S css\right)\right)$$

Finally, it has been noted that it may be possible to omit the term $(1/R_A)(V_R rs + R_T I_{Sc} ss)$ as this term has been observed to be negligible in data considered to date. Thus minimal impedance is observed. In this event, a scrubber which only worked upon the $C_A d/dt (V_R rs + R_T I_S css)$ components would likely perform almost as well. It is noted that $C_A$ is likely a constant for any given implant.

Overall the modified scrubber assessed works well, and has too many degrees of freedom, which presents the opportunity to undertake further optimisation.

Yet another embodiment is a Complex Pole Scrubber. If we assume that the artefact is a second order response (a double exponential is a subset of this kind of response), then we can estimate the parameters of the second order response that fits the raw signal. For discrete signals, the artefact g follows the model:

$$g[n] = b \cdot g[n-1] + c \cdot g[n-2]$$

Given a sequence of samples we may write down the matrix equation:

$$\vec{g} = A \begin{pmatrix} b \\ c \end{pmatrix}$$

where,

$$\vec{g} = \begin{pmatrix} g[n] \\ g[n-1] \\ \vdots \end{pmatrix} \quad , \quad A = \begin{pmatrix} g[n-1] & g[n-2] \\ g[n-2] & g[n-3] \\ \vdots & \vdots \end{pmatrix}$$

The coefficients b and c may therefore be determined by computing:

$$\begin{pmatrix} b \\ c \end{pmatrix} = (A^T A)^{-1} A^T \vec{g}$$

The invention is as defined in the appended claims.

**Claims**

1. A system (192) for separating a compound action potential from an artefact in a neural recording, the system comprising:

   a memory storing a set of basis functions comprising at least one compound action potential basis function and at least one artefact basis function;
   an input for receiving a neural recording of electrical activity in neural tissue; and
   a processor configured to decompose the neural recording by determining at least one of a compound action potential and an artefact using the set of basis functions, and further configured to output an estimate of at least one of a compound action potential and an artefact.

2. The system of claim 1 wherein the electrical activity comprises an evoked compound action potential, evoked by an electrical stimulus applied to the neural tissue.

3. The system of claim 2 wherein the artefact basis functions are matched to electrical artefact known to be caused by one or more such electrical stimuli.

4. The system of any one of claims 1 to 3 wherein respective estimates of more than one underlying signal are output, the more than one underlying signal including the compound action potential of the neural recording and the artefact of the neural recording.

5. The system of claim 4 wherein the more than one underlying signal further includes one or more of: background neuronal activity; and an evoked late response.

6. The system of any one of claims 1 to 5 wherein both an evoked compound action potential (ECAP) and an artefact are simultaneously estimated.

7. The system of any one of claims 1 to 6 wherein an estimate of a noiseless ECAP is output.

8. The system of any one of claims 1 to 7 wherein separating the compound action potential from the artefact in the neural recording is computationally efficient, to order O(n).

9. The system of any one of claims 1 to 8 wherein a computational process for separating the compound action potential

16

from the artefact in the neural recording is executed in a deterministic time.

10. The system of any one of claims 1 to 9 wherein a computational process for separating the compound action potential from the artefact in the neural recording is implemented in firmware of an implanted device.

11. The system of any one of claims 1 to 10 wherein each underlying signal is represented as a linear combination of basis functions.

12. The system of claim 6 wherein the ECAP is modelled in the basis functions by a function matched to an expected noiseless ECAP morphology.

13. The system of claim 12 wherein the ECAP is modelled in the basis functions by a piecewise function composed of one period of a sine wave followed by an exponential function such that the derivative is continuous at their boundary.

14. The system of claim 12 or claim 13 wherein the ECAP is modelled in the basis functions by two or more basis functions.

15. The system of claim 14 wherein the two more basis functions include a first basis function optimised for single ended neural recordings and a second basis function optimised for differential neural recordings.

16. The system of any one of claims 1 to 15 wherein artefact is modelled in the basis functions by three basis functions, comprising a DC basis function, a linear basis function, and an exponential basis function.

17. The system of any one of claims 1 to 15 wherein artefact is modelled in the basis functions by three basis functions, comprising a DC basis function, a linear basis function, and a fractional pole function.

18. The system of any one of claims 1 to 17, further configured to detect recordings in which only artefact exists, without any ECAP.

19. A computer-implemented method for separating a compound action potential from an artefact in a neural recording, the method comprising:

> accessing a memory containing a set of basis functions comprising at least one compound action potential basis function and at least one artefact basis function;
> receiving a neural recording of electrical activity in neural tissue; and
> decomposing the neural recording by determining at least one of a compound action potential and an artefact from the set of basis functions, and
> outputting an estimate of at least one of a compound action potential and an artefact.

**Patentansprüche**

1. System (192) zum Trennen eines Summenaktionspotentials von einem Artefakt in einer neuralen Aufzeichnung, wobei das System aufweist:

> einen Speicher, der einen Satz von Basisfunktionen speichert, der mindestens eine Summenaktionspotentialbasisfunktion und mindestens eine Artefaktbasisfunktion aufweist;
> eine Eingabe zum Empfangen einer neuralen Aufzeichnung elektrischer Aktivität in Nervengewebe; und
> einen Prozessor, der dazu ausgelegt ist, die neurale Aufzeichnung durch Bestimmen von mindestens einem von einem Summenaktionspotenzial und einem Artefakt unter Verwendung des Satzes von Basisfunktionen zu zerlegen, und ferner dazu ausgelegt ist, eine Schätzung von mindestens einem von einem Summenaktionspotenzial und einem Artefakt auszugeben.

2. System nach Anspruch 1, wobei die elektrische Aktivität ein evoziertes Summenaktionspotenzial aufweist, das durch einen elektrischen Reiz evoziert wird, der an das Nervengewebe angelegt wird.

3. System nach Anspruch 2, bei dem die Artefaktbasisfunktionen mit elektrischen Artefakten abgestimmt werden, von denen bekannt ist, dass sie durch einen oder mehrere solcher elektrischen Reize verursacht werden.

4. System nach einem der Ansprüche 1 bis 3, wobei entsprechende Schätzungen mehr als eines zugrundeliegenden Signals ausgegeben werden, wobei das mehr als eine zugrundeliegende Signal das Summenaktionspotenzial der neuralen Aufzeichnung und das Artefakt der neuralen Aufzeichnung aufweist.

5. System nach Anspruch 4, wobei das mehr als eine zugrundeliegende Signal ferner eines oder mehrere aufweist von: neuraler Hintergrundaktivität; und einer evozierten späten Reaktion.

6. System nach einem der Ansprüche 1 bis 5, wobei sowohl ein evoziertes Summenaktionspotential (ECAP) als auch ein Artefakt gleichzeitig geschätzt werden.

7. System nach einem der Ansprüche 1 bis 6, wobei eine Schätzung eines rauschfreien ECAP ausgegeben wird.

8. System nach einem der Ansprüche 1 bis 7, wobei das Trennen des Summenaktionspotentials von dem Artefakt in der neuralen Aufzeichnung rechnerisch effizient ist bis zur Größenordnung O(n).

9. System nach einem der Ansprüche 1 bis 8, wobei ein Rechenprozess zum Trennen des Summenaktionspotenzials von dem Artefakt in der neuralen Aufzeichnung in einer deterministischen Zeit ausgeführt wird.

10. System nach einem der Ansprüche 1 bis 9, wobei ein Rechenprozess zum Trennen des Summenaktionspotenzials von dem Artefakt in der neuralen Aufzeichnung in Firmware einer implantierten Vorrichtung implementiert ist.

11. System nach einem der Ansprüche 1 bis 10, wobei jedes zugrundeliegende Signal als Linearkombination von Basisfunktionen dargestellt wird.

12. System nach Anspruch 6, wobei das ECAP in den Basisfunktionen durch eine Funktion modelliert wird, die mit einer erwarteten rauschfreien ECAP-Morphologie abgestimmt ist.

13. System nach Anspruch 12, wobei das ECAP in den Basisfunktionen durch eine stückweise Funktion modelliert wird, die aus einer Periode einer Sinuswelle, gefolgt von einer Exponentialfunktion besteht, so dass die Ableitung an ihrer Grenze kontinuierlich ist.

14. System nach Anspruch 12 oder 13, wobei das ECAP in den Basisfunktionen durch zwei oder mehr Basisfunktionen modelliert wird.

15. System nach Anspruch 14, wobei die zwei weiteren Basisfunktionen eine erste Basisfunktion, die für einseitige neurale Aufzeichnungen optimiert ist, und eine zweite Basisfunktion, die für differentielle neurale Aufzeichnungen optimiert ist, aufweisen.

16. System nach einem der Ansprüche 1 bis 15, wobei das Artefakt in den Basisfunktionen durch drei Basisfunktionen modelliert wird, die eine DC-Basisfunktion, eine lineare Basisfunktion und eine exponentielle Basisfunktion aufweisen.

17. System nach einem der Ansprüche 1 bis 15, wobei das Artefakt in den Basisfunktionen durch drei Basisfunktionen modelliert wird, die eine DC-Basisfunktion, eine lineare Basisfunktion und eine fraktionierte Polfunktion aufweisen.

18. System nach einem der Ansprüche 1 bis 17, das ferner dazu ausgelegt ist, Aufzeichnungen zu erfassen, in denen nur Artefakte vorhanden sind, ohne ein ECAP.

19. Computerimplementiertes Verfahren zum Trennen eines Summenaktionspotentials von einem Artefakt in einer neuralen Aufzeichnung, wobei das Verfahren aufweist:

Zugreifen auf einen Speicher, der einen Satz von Basisfunktionen aufweist, der mindestens eine Summenaktionspotentialbasisfunktion und mindestens eine Artefaktbasisfunktion aufweist;
Empfangen einer neuralen Aufzeichnung elektrischer Aktivität in Nervengewebe; und
Zerlegen der neuralen Aufzeichnung durch Bestimmen von einem von einem Summenaktionspotenzial und einem Artefakt aus dem Satz von Basisfunktionen, und
Ausgeben einer Schätzung von mindestens einem von einem Summenaktionspotenzial und einem Artefakt.

**Revendications**

1. Système (192) de séparation d'un potentiel d'action composite d'un artéfact dans un enregistrement neuronal, le système comprenant :

   une mémoire qui enregistre un ensemble de fonctions de base comprenant au moins une fonction de base de potentiel d'action composite et au moins une fonction de base d'artéfact ;
   une entrée pour recevoir un enregistrement neuronal d'activité électrique dans un tissu neuronal ; et
   un processeur configuré pour décomposer l'enregistrement neuronal en déterminant au moins l'un parmi un potentiel d'action composite et un artéfact en utilisant un ensemble de fonctions de base, et configuré en outre pour produire une estimation d'au moins un parmi un potentiel d'action composite et un artéfact.

2. Système selon la revendication 1, dans lequel l'activité électrique comprend un potentiel d'action composite provoqué, provoqué par un stimulus électrique appliqué au tissu neuronal.

3. Système selon la revendication 2, dans lequel les fonctions de base d'artéfact sont associées à un artéfact électrique connu pour être causé par au moins un tel stimulus électrique.

4. Système selon l'une quelconque des revendications 1 à 3, dans lequel sont produites des estimations respectives de plusieurs signaux fondamentaux, les plusieurs signaux fondamentaux comprenant le potentiel d'action composite de l'enregistrement neuronal et l'artéfact de l'enregistrement neuronal.

5. Système selon la revendication 4, dans lequel les plusieurs signaux fondamentaux comprennent en outre au moins un parmi : une activité neuronale en fond ; et une réaction tardive provoquée.

6. Système selon l'une quelconque des revendications 1 à 5, dans lequel sont estimés à la fois un potentiel d'action de composite provoqué (ECAP) et un artéfact.

7. Système selon l'une quelconque des revendications 1 à 6, dans lequel est produit une estimation d'ECAP sans bruit de fond.

8. Système selon l'une quelconque des revendications 1 à 7, dans lequel séparer le potentiel d'action composite de l'artéfact dans l'enregistrement neuronal est efficace informatiquement à l'ordre O(n).

9. Système selon l'une quelconque des revendications 1 à 8, dans lequel un processus informatique de séparation du potentiel d'action composite de l'artéfact dans l'enregistrement neuronal est effectué à un moment déterministe.

10. Système selon l'une quelconque des revendications 1 à 9, dans lequel un processus informatique de séparation du potentiel d'action composite de l'artéfact dans l'enregistrement neuronal est mis en œuvre dans un logiciel d'un dispositif implanté.

11. Système selon l'une quelconque des revendications 1 à 10, dans lequel chacun des signaux fondamentaux est représenté comme une combinaison linéaire de fonctions de base.

12. Système selon la revendication 6, dans lequel l'ECAP est modélisé dans les fonctions de base par une fonction associée à une morphologie attendue d'ECAP sans bruit de fond.

13. Système selon la revendication 12, dans lequel l'ECAP est modélisé dans les fonctions de base par une fonction par morceaux constituée d'une période d'une fonction sinusoïdale suivie d'une fonction exponentielle, de sorte que sa dérivée soit continue à leur point limite.

14. Système selon la revendication 12 ou la revendication 13, dans lequel l'ECAP est modélisée dans les fonctions de base par au moins deux fonctions de base.

15. Système selon la revendication 14, dans lequel les deux fonctions de base de plus comportent une première fonction de base optimisée pour des enregistrements neuronaux single-ended et une seconde fonction de base optimisée pour des enregistrements neuronaux différentiels.

**16.** Système selon l'une quelconque des revendications 1 à 15, dans lequel l'artéfact est modélisé dans les fonctions de base par trois fonctions de base comprenant une fonction de base DC, une fonction de base linéaire et une fonction de base exponentielle.

**17.** Système selon l'une quelconque des revendications 1 à 15, dans lequel l'artéfact est modélisé dans les fonctions de base par trois fonctions de base comprenant une fonction de base DC, une fonction de base linéaire et une fonction à pôle fractionnel.

**18.** Système selon l'une quelconque des revendications 1 à 17, configuré en outre pour détecter des enregistrements dans lesquels seul existe l'artéfact sans aucun ECAP.

**19.** Méthode mise en œuvre par ordinateur pour séparer un potentiel d'action composite d'un artéfact dans un enregistrement neuronal, la méthode comprenant de :

accéder à une mémoire contenant un ensemble de fonctions de base comprenant au moins une fonction de base de potentiel d'action composite et au moins une fonction de base d'artéfact ;
recevoir un enregistrement neuronal d'activité électrique dans un tissu neuronal ; et
décomposer l'enregistrement neuronal en déterminant au moins un parmi un potentiel d'action composite et un artéfact dans l'ensemble de fonctions de base, et
produire une estimation d'au moins un parmi un potentiel d'action composite et un artéfact.

**Figure 1**

**Figure 2**

**Figure 3**

**Fig. 4**

Signal: $\sigma$

Feedback mechanism $\underline{510}$

502 → Preprocessing   Basis A   Basis B

504 → Basis element separation

506 → Post-processing   Post-processing

eCAP: $f$   Artefact: $g$

**Fig. 5**

Piecewise Sine Component   Gamma PDF   Generic ECAP Model

**Fig. 6**

**Figure 7**

**Figure 8**

**Figure 9**

**Figure 10**

**Figure 11**

**Figure 12**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 9044155 B
- WO 2018170141 A
- AU 2019051151 W
- WO 2012155183 A
- WO 2015070281 A
- AU 2019051160 W

### Non-patent literature cited in the description

- **ISLAM MD KAFIUL et al.** Methods for artifact detection and removal from scalp EEG: A review. *NEUROPHYSIOLOGIE CLINIQUE - CLINICAL NEUROPHYSIOLOGY*, vol. 46 (4), 287-305